# EUROPEAN PATENT SPECIFICATION

(11) **EP 4 192 336 B1**
(45) Date of publication and mention of the grant of the patent: **16.09.2026**
(21) Application number: 21763134.0
(22) Date of filing: 06.08.2021
(51) Int. Cl.: A61B 5/00, A61B 5/16, A61B 5/11

(54) **SYSTEM FOR OVERCOMING FOG (FREEZING OF GAIT) EPISODES**
SYSTEM ZUR ÜBERWINDUNG VON "FOG" (FREEZING-OF-GAIT, EINFRIEREN DES GANGS) EPISODEN
SYSTÈME POUR SURMONTER DES ÉPISODES DE "FOG" (FREEZING-OF-GAIT, BLOCAGE DE LA MARCHE)

(30) Priority: 07.08.2020 IT 202000019825
(43) Date of publication of application: 14.06.2023
(73) Proprietor: Mhealth Technologies S.r.l., 40050 Monte San Pietro (BO) (IT); Schema31 S.p.A., 00154 Roma (RM) (IT)
(72) Inventor: TACCONI, Carlo, 40127 Bologna (BO) (IT); PALMERINI, Luca, 40127 Bologna (BO) (IT); PICCININI, Roberta, 40127 Bologna (BO) (IT); FERRARI, Alberto, 40127 Bologna (BO) (IT); MANZI, Salvatore, 00154 Roma (RM) (IT); BERTOCCI, Paolo, 00154 Roma (RM) (IT); ZACCHEO, Fabrizio, 00154 Roma (RM) (IT); CAMPIGLI, Luigi, 50054 Fucecchio (FI) (IT); TESCONI, Mario, 55100 Lucca (LU) (IT); ZUPONE, Giuseppe, 55100 Lucca (LU) (IT); SGAMBELLURI, Nicola, 55100 Lucca (LU) (IT); MELLONE, Sabato, 40127 Bologna (BO) (IT)
(74) Representative: Zoli, Filippo
(86) International application number: PCT/IB2021/057263
(87) International publication number: WO 2022/029708

(56) References cited:
- EP-A1- 2 335 231
- EP-B1- 2 335 231
- WO-A1-2014/032181
- US-A1- 2014 276 130
- US-A1- 2018 132 758
- DJURIC-JOVICIC MILICA D ET AL: "Automatic Identification and Classification of Freezing of Gait Episodes in Parkinson's Disease Patients", IEEE TRANSACTIONS ON NEURAL SYSTEMS AND REHABILITATION ENGINEERING, IEEE SERVICE CENTER, NEW YORK, NY, US, vol. 22, no. 3, 1 May 2014 (2014-05-01), pages 685 - 694, XP011546780, ISSN: 1534-4320, [retrieved on 20140428], DOI: 10.1109/TNSRE.2013.2287241

## Description

### Technical Field

The present invention relates to a system for overcoming FOG (freezing of gait) episodes.

### Background Art

FOG is a symptom generally experienced by people with Parkinson's disease that causes moments of involuntary gait interruption or blockage. These interruptions can last up to several seconds, thus jeopardizing the mobility of these individuals.

Over the years, a number of solutions have been provided using sensory stimuli, such as e.g. audio or visual signals, which aim to stimulate specific areas of the brain in order to bypass the brain areas affected by the blockage and reduce the effects of FOG episodes on the affected person.

To date, these solutions have relied on generic motor correction systems which employ sensory stimulus emission means wearable by the user and adapted to subject the user to such stimulus continuously or periodically.

For example, motor correction systems are known which are provided with light signal emitters, such as e.g. laser emitters, LEDs or the like, adapted to project an image, such as a line of light, onto the ground along the path followed by the user.

Such emitters are generally wearable, e.g. they can be mounted on the user's footwear or they can be installed on a walking stick used by the user for walking.

Other emitters coincide with augmented reality glasses which allow an image to be displayed directly on the lenses of the glasses themselves.

Some systems are also known which use socks provided with vibration emitters which are adapted to stimulate the user's foot by means of vibrating impulses. This type of system allows reducing the effects of FOG episodes on gait. However, such systems subject the user to continuous exposure to sensory stimulation, even in the absence of FOG episodes.

To overcome this drawback, systems employing appropriately configured detection means for detecting the occurrence of FOG episodes are known from patent documents WO2010124013A1 and WO2019086997A2.

These detection means are generally mounted on footwear worn by the subject suffering from FOG and configured to signal the detection of an FOG episode to the emission means, which, this way, emit the stimulus which allows the subject to overcome FOG, i.e. the motor block, and resume normal gait.

In particular, these systems avail themselves of detection means provided with sensor means configured to detect data inherent in the movement of the footwear and processing means for processing such data, configured to signal to the emission means the detection, and therefore the occurrence, of the FOG episode in the subject.

Even these types of systems are however susceptible to improvements in their degree of reliability and effectiveness.

Indeed, very frequently the detection means employed by the systems of known type misinterpret the data acquired by the acquisition means and cause the emission of the sensory stimulus even when no FOG episode has occurred.

In addition, the detection means used by this type of system is particularly prone to malfunction and breakage, since, being mounted on the footwear, they are subject to shock and vibration including over long periods of time. Furthermore, the detection means and the acquisition means are made as a single electronic device which is particularly complex, expensive and inconvenient to use.

These drawbacks make known systems unreliable, expensive and frequently subject to maintenance/repair, and they are uncomfortable and inconvenient to use.

Other systems for overcoming fog (freezing of gait) episodes are known from WO 2014/032181 A1, EP 2 335 231 A1, US2018/132758 A1, US 2014/276130 A1 and XP011546780.

### Description of the Invention

The main object of the present invention is to devise a system for overcoming FOG episodes which allows for increasing the reliability of the system with respect to those of known type.

An additional object of the present invention is to devise a system for overcoming FOG episodes which allows detecting with more accuracy the occurrence of an FOG episode with respect to the systems of known type.

An additional object of the present invention is to devise a system for overcoming FOG episodes which allows reducing the impact of shock and vibrations on the detection means.

Still one object of the present invention is to devise a system for overcoming FOG episodes which allows simplifying the system itself with respect to those of known type.

An additional object of the present invention is to devise a system for overcoming FOG episodes which allows increasing the flexibility in the use of the system itself with respect to those of known type.

An additional object of the present invention is to devise a system for overcoming FOG episodes which triggers only in the event that the user of the system is subjected to an FOG episode.

Another object of the present invention is to devise a system for overcoming FOG episodes which allows overcoming the drawbacks mentioned above of the prior art within the scope of a simple, rational, easy and effective to use as well as affordable solution.

The objects set out above are achieved by the present a system for overcoming FOG episodes having the characteristics of claim 1.

### Brief Description of the Drawings

Other characteristics and advantages of the present invention will be more apparent from the description of several preferred, but not exclusive embodiments of a system for overcoming FOG episodes, illustrated by way of an indicative, yet non-limiting example, in the attached tables of drawings wherein:
Figures 1-4 are schematic views of an embodiment of the system 1 according to the invention;

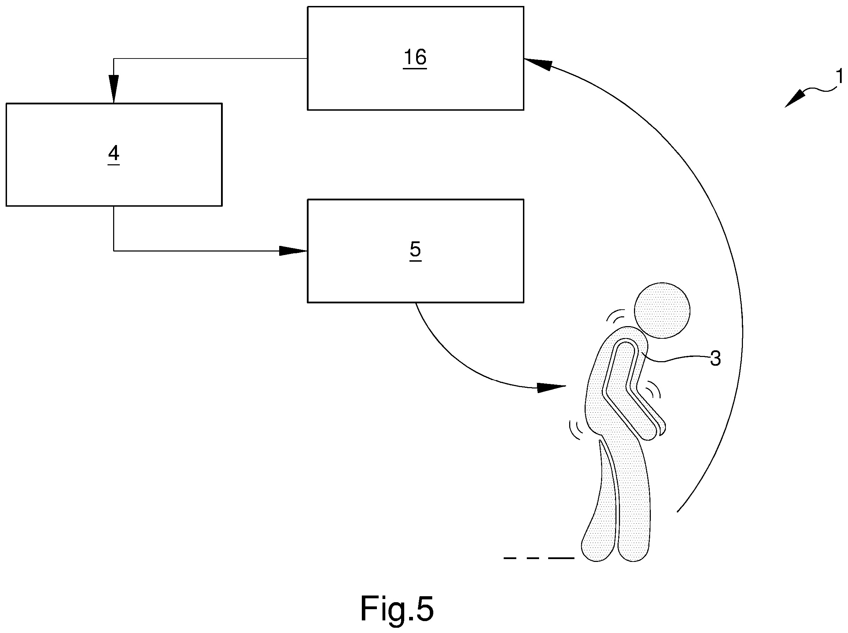

Figure 5 is a schematic view of a further embodiment of the system according to the invention;
Figure 6 is a schematic view of an additional embodiment of the system according to the invention;
Figure 7 is a schematic view of an additional embodiment of the system according to the invention;
Figure 8 is a schematic view of another embodiment of the system according to the invention;
Figures 9 to 11 are schematic views of an additional embodiment of the system according to the invention.

### Embodiments of the Invention

With particular reference to these figures, reference numeral 1 globally indicates a system for overcoming FOG episodes.

The system 1 for overcoming FOG (Freezing of Gait) episodes comprises:
- acquisition means 16 of at least one FOG-related motor datum of a subject 3 affected by FOG episodes, the acquisition means 16 being mounted on at least one piece of footwear 7a, 7b worn by the subject 3;
- detection means 4, separate from the acquisition means 16, operationally connected to the latter and configured to detect at least one FOG episode depending on at least the detected motor datum;
- emission means 5 for emitting at least one sensory stimulus perceivable by the subject 3 as a result of the detection of the FOG episode and which allows the subject 3 to overcome such an FOG episode, worn by the subject 3, and operationally connected to the detection means 4, the latter being configured to signal to the emission means 5 the detection of the FOG episode.

The term FOG is intended to indicate an involuntary blockage or interruption of gait for a few seconds, such as e.g. an inability to take a step.

Conveniently, the acquisition means 16 comprise at least one acquisition assembly 2a, 2b provided with at least one sensor device 6 of the type selected from the list comprising a gyroscope, an accelerometer, a speedometer, a position locator, a pressure transducer, a magnetometer, and configured to acquire the at least one motor datum.

In particular, the acquisition assembly 2a, 2b is configured to transmit at least the acquired motor datum to the detection means 4.

Moreover, the acquisition assembly 2a, 2b comprises a plurality of sensor devices 6.

Preferably, the acquisition assembly 2a, 2b comprises at least one gyroscope, at least one accelerometer, at least one speed meter, at least one position locator, at least one pressure transducer and at least one magnetometer.

Advantageously, the acquisition assembly 2a, 2b comprises at least one gyroscope, at least one accelerometer and at least one pressure transducer.

This way, the system 1 is able to accurately determine the state of motion of the footwear 7a, 7b on which the acquisition assembly 2a, 2b is mounted. Furthermore, the acquisition assembly 2a, 2b comprises at least one rechargeable battery, not shown in the illustrations, configured to electrically power the same acquisition assembly 2a, 2b.

Conveniently, the acquisition assembly 2a, 2b comprises at least one data filing unit 8 configured to store the acquired motor data.

Advantageously, the sensor devices 6 are mounted on the footwear 7a, 7b at predefined positions.

Preferably, the acquisition assembly 2a, 2b comprises at least one sensor device 6 arranged at at least one of either the heel or the toe of the footwear 7a, 7b. Alternatively or additionally, the acquisition assembly 2a, 2b comprises at least one sensor device 6 positioned between the heel and the toe of the footwear 7a, 7b.

According to a possible embodiment of the system 1, the system itself comprises a plurality of sensor devices 6 of the type of pressure transducers, arranged at respective predefined positions of the footwear 7a, 7b intended to support the weight/pressure exerted on the footwear itself by a relevant portion of the sole of the foot, and at least one sensor device 6 of the type of a motion sensor, such as e.g., a gyroscope.

Conveniently, the acquisition means 16 comprise a pair of acquisition assemblies 2a, 2b, each of which is mounted on a corresponding footwear 7a, 7b worn by the subject 3.

In this way, each acquisition assembly 2a, 2b is configured to acquire at least one motor datum related to the footwear 7a, 7b on which it is mounted.

This expedient allows the system 1 to accurately determine the state of motion of each piece of footwear 7a, 7b, so as to accurately detect the occurrence of an FOG episode, thus reducing the risk of detecting false FOG episodes. Advantageously, the detection means 4 comprise at least one processing unit 9 of at least one motor characteristic processed depending on at least one motor datum.

Furthermore, the detection means 4 are configured to:
- compare at least one predefined reference value with at least one reference value attributed to or derived from one or more motor characteristics depending on at least the value of the latter;
- detect at least one FOG episode as a result of the comparison between at least the attributed or derived reference value and the predefined reference value.

In particular, each of the motor characteristics processed by the processing unit 9 is defined or derived from at least one measurable physical quantity. Appropriately, the term reference value is not intended to indicate only a single value; in fact, the term is intended to indicate, e.g., also a set of values defining one or more reference ranges.

Preferably, the reference value or values are expressed in terms of probability.

Advantageously, the detection means 4 are configured to relate the attributed or derived reference value (generally expressed by means of at least one physical quantity) of at least one of the processed motor characteristics to at least one corresponding predefined reference value.

In particular, the detection means 4 are configured to:
- process/provide the predefined reference value depending on a motor characteristic or multiple combined motor characteristics taken into account to detect the occurrence of at least one FOG episode;
- attribute to or derive from the motor characteristic or combination of motor characteristics taken into account to detect the occurrence of at least one FOG episode of at least the attributed or derived reference value.

More in detail, the detection means 4 are configured to process/provide the predefined reference value depending on the type of one motor characteristic or multiple motor characteristics combined to each other taken into account to detect the occurrence of at least one FOG episode.

A list of the type of motor characteristics is provided below.

Conveniently, the detection means 4 are configured to report the occurrence of an FOG episode when the reference value attributed to or derived from a motor characteristic, or when the combination of reference values attributed to or derived from different motor characteristics, belongs to the predefined reference value.

In particular, according to the invention, the detection means 4 are configured to attribute to or derive from a linear combination of a plurality of motor characteristics the attributed or derived reference value.

For example, the detection means 4 are configured to attribute or derive, depending on a non-linear combination of K (k meant as an arbitrary integer number) motor characteristics, the reference value attributed to or derived from each or the combination of the K motor characteristics. The type of such K motor characteristics defines a predefined space inside which one or more regions are identified wherein the observed event is detected as FOG, i.e., the predefined reference value. Accordingly, the detection means 4 are configured to compare the attributed or derived reference value and the predefined reference value, and to report the occurrence of an FOG episode if the attributed or derived reference value belongs to a region of the predefined space.

In other words, the detection means 4 are configured to identify a predefined reference value depending on each type or each combination of types of motor characteristics taken into account to detect the occurrence of an FOG episode. Furthermore, the detection means 4 are configured to compare the predefined reference value with the attributed or derived reference value of the motor characteristic taken into account to detect the occurrence of an FOG episode or with a combination of attributed or derived reference values of a combination of motor characteristics taken into account to detect the occurrence of an FOG episode.

Conveniently, the system 1 comprises indication means, not shown in the figures, for indicating the type(s) of motor characteristics to be taken into account to detect the occurrence of an FOG episode.

Furthermore, the indication means are configured to indicate the number of motor characteristics to be taken into account to detect the occurrence of an FOG episode.

In this way, unlike systems which carry out a simple gait analysis, the detection means 4 allow the particularly specific and precise detection of the occurrence of an FOG episode.

Preferably, the processing unit 9 is configured to process at least one motor characteristic of the type of an instantaneous motor characteristic selected from the list comprising at least one of:
- the linear velocity of at least one piece of footwear 7a, 7b;
- the angular velocity of at least one piece of footwear 7a, 7b;
- frequency content of the angular velocity signal of at least one piece of footwear 7a, 7b;
- modulus of the angular velocity signal of at least one piece of footwear 7a, 7b;
- signal strength in predefined frequency bands for the angular velocity signal of at least one piece of footwear 7a, 7b;
- linear acceleration of at least one piece of footwear 7a, 7b;
- frequency content of the linear acceleration signal of at least one piece of footwear 7a, 7b;
- modulus of the linear acceleration signal of at least one piece of footwear 7a, 7b;
- signal strength in predefined frequency bands for the linear acceleration signal of at least one piece of footwear 7a, 7b;
- the angular acceleration of at least one piece of footwear 7a, 7b;
- the orientation in space of at least one piece of footwear 7a, 7b;
- tremor of at least one piece of footwear 7a, 7b;
- map of the plantar pressure of at least one piece of footwear 7a, 7b;
- modulus of the pressure map of at least one piece of footwear 7a, 7b;
- strength of the pressure map in predefined frequency bands for the linear acceleration signal of at least one piece of footwear 7a, 7b;
- frequency content of the pressure map of at least one piece of footwear 7a, 7b;
- the step cadence of at least one piece of footwear 7a, 7b;
- the stride length of at least one piece of footwear 7a, 7b;
- the stride duration of at least one piece of footwear 7a, 7b;
- asymmetry of the stride of one piece of footwear 7a, 7b, with respect to another piece of footwear 7a, 7b;
- pitch contact of at least one piece of footwear 7a, 7b;
- pitch cycle of at least one piece of footwear 7a, 7b;
- dyskinesia of at least one piece of footwear 7a, 7b;
- the distance covered by the subject 3;
- the number of steps of at least one piece of footwear 7a, 7b.

Preferably, one or more of the motor characteristics are expressed as signals.

Furthermore, the processing unit 9 is configured to process at least one motor characteristic of the type of the at least one variable motor characteristic, i.e., the variation of one of the instantaneous motor characteristics at different instants of time. For example, a variable motor characteristic comprises the variability of the speed of at least one piece of footwear 7a, 7b at different instants of time.

The term gait asymmetry is meant to indicate the difference between motor data acquired by the acquisition assemblies 2a, 2b, such as e.g. the difference between the speed of the two pieces of footwear 7a, 7b.

The term pitch contact is meant to indicate the angle of incidence of the piece of footwear 7a, 7b with the ground the moment it rests on the ground.

The term pitch cycle is meant to indicate the state of the piece of footwear 7a, 7b, e.g. if the same is arranged totally resting on the ground, if it is arranged partly resting on the ground during the lifting phase or during the resting phase on the ground.

In particular, the detection means 4 are configured to signal to the emission means 5 the occurrence of the detected FOG episode.

Appropriately, the detection means 4 comprise a storage unit 10 configured to store the motor data received from the acquisition means 16.

Advantageously, the storage unit 10 is configured to store the motor characteristics processed by the processing unit 9.

Furthermore, the storage unit 10 is configured to store data significant to the detection of the FOG event, such as e.g., but not limited to, the date and time of detection of an FOG event.

Preferably, the detection means 4 are of the type of an electronic device wearable by the subject 3, such as e.g. a smartphone, smartwatch, headset device, smartglasses, belt, wristband and the like.

Furthermore, the detection means 4 comprise at least one rechargeable battery, not shown in the illustrations, configured to electrically power the detection means themselves.

Advantageously, the emission means 5 are configured to emit a sensory stimulus of the type selected from the list comprising a visual, auditory, vibrotactile, thermal, olfactory, taste stimulation.

Preferably, auditory stimulation may be via emission means 5 of the type of headphones, speakers or bone transducers.

Visual stimulation, on the other hand, may take place via emission means 5 of the type of smartglasses or image projectors onto the ground.

Vibrotactile, thermal or olfactory stimulation may preferably take place via emission means 5 of the type of vibrating, thermal devices or through the release of particular essences, to be worn at points sensitive to such stimulation such as, e.g., on the wrist or on the waist of the subject 3.

Alternative embodiments of the system 1 cannot be ruled out wherein the emission means 5 are of the type of an electronic device of the type of a smartphone, a smartwatch, a headset device, smartglasses or of the type of an electronic device integrating with a wearable accessory, e.g., a belt, a bracelet and the like.

Preferably, the emission means 5 are configured to emit the sensory stimulus for a predefined time.

Furthermore, the emission means 5 are configured to emit the sensory stimulus with a predefined frequency.

**In** addition, the emission means 5 are configured to emit the sensory stimulus with a predefined intensity.

**In** particular, the emission means 5 are configured to emit at least one sensory stimulus as a result of the detection of an FOG event.

**In** fact, the emission means 5 are configured to receive from the detection means 4 reports of the detection of FOG events.

Conveniently, the system 1 comprises command means, not shown on the illustrations, operationally connected to at least one of the acquisition means 16, the detection means 4 or the emission means 5, and configured to be used by at least one user to:
- indicate at least one of:
   - the type of sensory stimulus to be emitted;
   - the duration of the emitted sensory stimulus;
   - the frequency of the emitted sensory stimulus;
   - the intensity of the stimulus;
- and/or to display at least one of:
- at least one of the motor characteristics;
- at least one of the motor data.

Preferably, the command means comprise at least one of either:
- user interface means wearable by the subject 3;
- remote control means usable by at least one user.

The user interface means are preferably of the type of a smartphone, smartwatch, tablet, PC or the like.

Alternative embodiments of the system 1 cannot however be ruled out wherein the user interface means coincide with at least one of either the detection means 4 or the emission means 5.

The remote control means are preferably of the type of any electronic control device which employs a wireless communication network, such as e.g., the Internet, to remotely control the system 1.

Conveniently, the system 1 comprises connection means, not shown in the illustrations, of at least one of the acquisition means 16, the detection means 4, the emission means 5 and the command means with at least another of the means selected from the same list.

In particular, the connection means are configured to allow the exchange of information, e.g., data, signals and the like, between the means connected to each other.

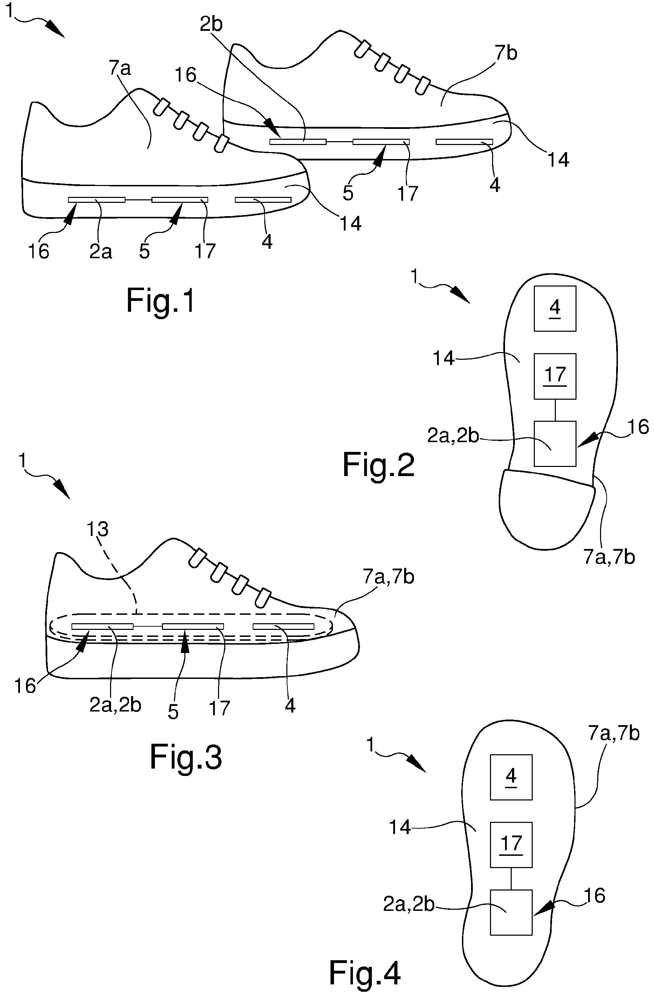

Preferably, the detection means 4 are mounted on the footwear 7a, 7b. According to the embodiment of the system 1 shown in figures 1 to 4, the detection means 4 and the emission means 5 are mounted on the footwear 7a, 7b.

Preferably, in this embodiment the emission means 5 comprise a pair of emission assemblies 17, each of which is mounted on a corresponding piece of footwear 7a, 7b worn by the subject 3.

Suitably, each emission assembly 17 is configured to emit a sensory stimulus. In this way, the subject 3 may be selectively stimulated by the stimulus emitted by at least one of the emission assemblies 17.

Preferably, the system 1 comprises at least one of either:
- at least one piece of footwear 7a, 7b worn by the subject 3; or
- at least one insole 13 insertable inside a corresponding piece of footwear 7a, 7b worn by the subject 3, at least one of either the acquisition means 16, the detection means 4 or the emission means 5 being mounted on the insole 13.

In particular, according to a possible embodiment of the system 1, at least one of the acquisition means 16, the detection means 4 or the emission means 5 are integrated with the footwear 7a, 7b. In this way, the subject 3 wears at least one of the acquisition means 16, the detection means 4 or the emission means 5 when wearing the footwear 7a, 7b.

Preferably, in this embodiment of the system 1 at least one of either the acquisition means 16, the detection means 4 or the emission means 5 are integrated inside the outsole 14 of the footwear 7a, 7b.

Furthermore, preferably, the system 1 comprises a pair of footwear 7a, 7b.

According to a further possible embodiment of the system 1, at least one of the acquisition means 16, the detection means 4 or the emission means 5 are integrated with the insole 13. This way, the subject 3 places the insole 13 inside the footwear 7a, 7b which he or she wishes to wear in order to wear at least one of either the acquisition means 16, the detection means 4 or the emission means 5.

Preferably, the acquisition means 16 are integrated with the insole 13.

In particular, the system 1 comprises a pair of insoles 13, each insertable inside a corresponding piece of footwear 7a, 7b.

Further embodiments of the system 1 cannot however be ruled out, wherein at least one of either the acquisition means 16, the detection means 4 or the emission means 5 are integrated with the footwear 7a, 7b and at least one of either the acquisition means 16, the detection means 4 or the emission means 5 are integrated with the insole 13.

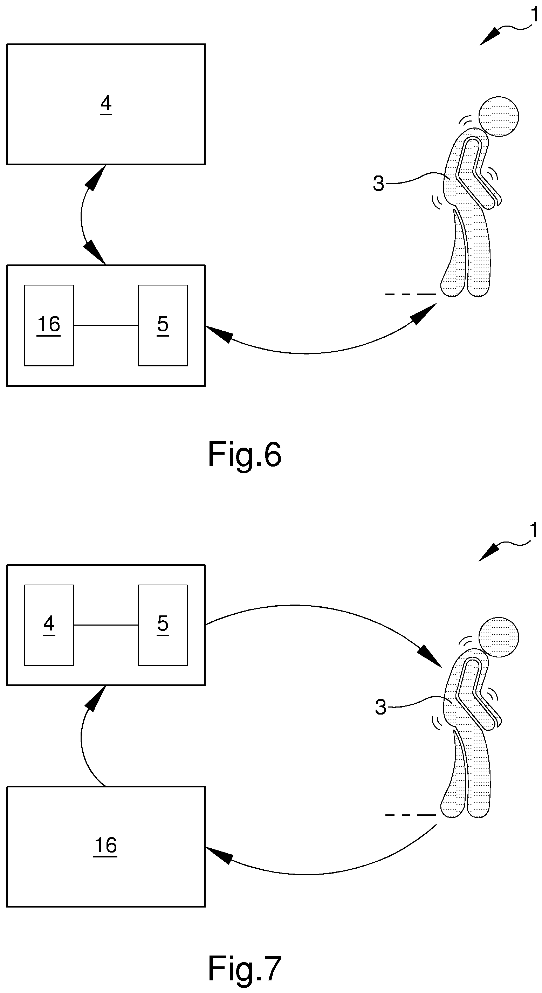

Further embodiments of the system 1 cannot however be ruled out, wherein the acquisition means 16 and the emission means 5 coincide with each other, as shown in Figure 6.

Preferably, in this embodiment the acquisition means 16 and the emission means 5 define a pair of electronic devices, each of which is mounted on a respective piece of footwear 7a, 7b and is configured to acquire at least one motor datum and emit at least one sensory stimulus.

In an additional embodiment of the system 1 shown in Figure 7, the detection means 4 coincide with the emission means 5.

Preferably, in this embodiment the detection means 4 and the emission means 5 define a single electronic device separate from the acquisition means 16, wearable by the subject 3 and configured to detect at least one FOG episode and emit at least one sensory stimulus.

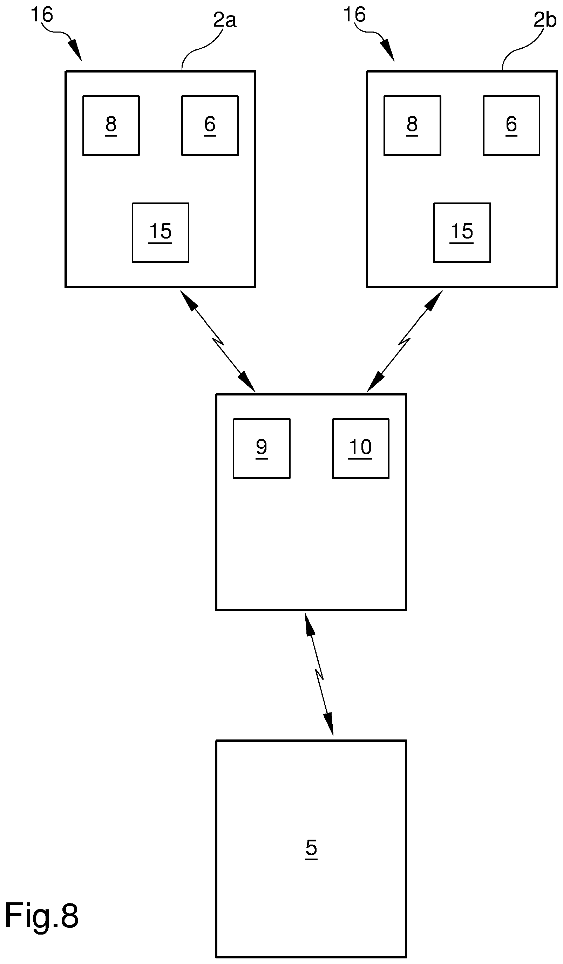

In a further embodiment of the system 1 shown in Figure 8, the system 1 comprises at least detection unit 15, mounted on at least one piece of footwear 7a, 7b, operationally connected to the acquisition assembly 2a, 2b and configured to detect at least one FOG episode depending on at least the motor datum.

Advantageously, everything which has been described with respect to the detection means 4 is also believed to apply to the detection unit 15.

In other words, the detection unit 15 is configured to detect the occurrence of an FOG episode in the same manner as the detection means 4.

Furthermore, in this embodiment the detection means 4 are configured to verify the FOG episode detected by the detection unit 15 to confirm the occurrence thereof and to signal to the emission means 5 the detection of the FOG episode as a result of this confirmation.

In this way, the emission means 5 emit the sensory stimulus as a result of verification and subsequent confirmation of the occurrence of the FOG episode. This solution makes it possible to reduce the risk of detecting false FOG episodes by reducing the risk of emitting a sensory stimulus even in the absence of an FOG episode.

Preferably, in this embodiment, the acquisition means 16 comprise an acquisition assembly 2a, 2b mounted on a first piece of footwear 7a, and an acquisition assembly 2a, 2b mounted on a second piece of footwear 7b. Furthermore, the system 1 comprises a pair of detection units 15 mounted on respective pieces of footwear 7a, 7b to detect the occurrence of at least one FOG episode depending on at least the motor datum related to the footwear 7a, 7b on which they are mounted.

In this way, the detection means 4 are configured to verify at least one of at least one FOG episode detected by the detection unit 15 related to the first piece of footwear 7a and at least one FOG episode detected by the detection unit 15 related to the second piece of footwear 7b to confirm the occurrence of the FOG episode and to signal to the emission means 5 the detection thereof as a result of this confirmation.

This solution allows further reducing the risk of detection of false FOG episodes by reducing the risk of emitting a sensory stimulus even in the absence of an FOG episode.

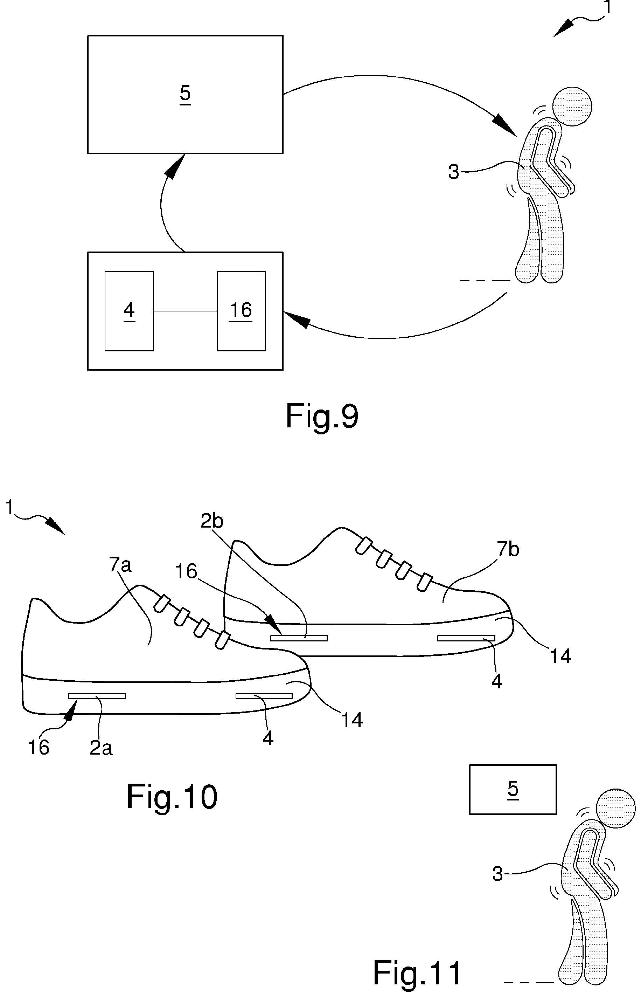

According to the embodiment of the system 1 shown in Figures 9-11, at least one piece of footwear 7a, 7b comprises at least one outsole 14 and the detection means 4 are associated with said outsole 14.

Furthermore, according to this embodiment, the acquisition assembly 2a, 2b is associated with the outsole 14 and comprises at least one sensor device 6 of the type selected from the list comprising a gyroscope, an accelerometer, a speedometer, a position locator, a magnetometer, and at least one sensor device 6 of the type of a pressure transducer.

In addition, according to this embodiment, the emission means 5 are worn on the upper part of the body of the subject 3 starting from the waistline of the same.

Preferably, at least one of either the acquisition assembly 2a, 2b or the detection means 4 are integrated with the piece of footwear 7a, 7b. In this embodiment, the subject 3 wears at least one of either the acquisition assembly 2a, 2b or the detection means 4 when wearing the piece of footwear 7a, 7b.

Preferably, in this embodiment the emission means 5 are of the type of a single separate electronic device worn by the subject 3 and not mounted on the piece of footwear 7a, 7b.

Further embodiments of the system 1 obtained by combining one or more characteristics of the above described embodiments cannot however be ruled out.

The present invention also relates to a method for overcoming FOG episodes. The method for overcoming FOG (Freezing of Gait) episodes comprises at least the phases of:
- acquisition of at least one FOG-related motor datum of a subject 3 affected by FOG episodes;
- detection of at least one FOG episode depending on at least the acquired motor datum;
- emission of at least one sensory stimulus perceivable by the subject 3 to overcome the FOG episode.

In particular, the detection phase comprises at least the steps of:
- processing of at least one processed motor characteristic depending on the at least one motor datum;
- processing/provision of a predefined reference parameter depending on a motor characteristic or multiple motor characteristics combined together taken into account to detect the occurrence of at least one FOG episode;
- attribution to or derivation from the motor characteristic or from the combination of motor characteristics taken into account to detect the occurrence of at least one FOG episode of at least one attributed or derived reference parameter depending on at least the value of the latter;
- comparison at least of the attributed or derived reference parameter with the predefined reference parameter;
- detection of the FOG episode as a result of the comparison.

In addition, the detection phase comprises at least one ascertainment step of the movement of the subject adapted to check whether the latter is moving. Advantageously, the ascertainment step is performed by means of at least one of the sensor devices 6, selected from a gyroscope, an accelerometer, a speedometer, a position locator and a magnetometer, and at least one sensor device 6 of the type of a pressure transducer.

Conveniently, the processing/provision phase of the predefined reference parameter processes/provides the predefined reference parameter depending on each type or each combination of types of motor characteristics taken into account to detect the occurrence of an FOG episode.

In particular, the attribution or derivation phase attributes or derives from a linear combination of a plurality of motor characteristics the attributed or derived reference parameter.

Conveniently, the processing step provides for the processing at least of a motor characteristic of the type of an instantaneous motor characteristic selected from the list comprising at least one of:
- the linear velocity of at least one piece of footwear 7a, 7b;
- the angular velocity of at least one piece of footwear 7a, 7b;
- frequency content of the angular velocity signal of at least one piece of footwear 7a, 7b;
- modulus of the angular velocity signal of at least one piece of footwear 7a, 7b;
- signal strength in predefined frequency bands for the angular velocity signal of at least one piece of footwear 7a, 7b;
- linear acceleration of at least one piece of footwear 7a, 7b;
- frequency content of the linear acceleration signal of at least one piece of footwear 7a, 7b;
- modulus of the linear acceleration signal of at least one piece of footwear 7a, 7b;
- signal strength in predefined frequency bands for the linear acceleration signal of at least one piece of footwear 7a, 7b;
- the angular acceleration of at least one piece of footwear 7a, 7b;
- the orientation in space of at least one piece of footwear 7a, 7b;
- tremor of at least one piece of footwear 7a, 7b;
- map of the plantar pressure of at least one piece of footwear 7a, 7b;
- modulus of the pressure map of at least one piece of footwear 7a, 7b;
- strength of the pressure map in predefined frequency bands for the linear acceleration signal of at least one piece of footwear 7a, 7b;
- frequency content of the pressure map of at least one piece of footwear 7a, 7b;
- the step cadence of at least one piece of footwear 7a, 7b;
- the stride length of at least one piece of footwear 7a, 7b;
- the stride duration of at least one piece of footwear 7a, 7b;
- asymmetry of the stride of one piece of footwear 7a, 7b, with respect to another piece of footwear 7a, 7b;
- pitch contact of at least one piece of footwear 7a, 7b;
- pitch cycle of at least one piece of footwear 7a, 7b;
- dyskinesia of at least one piece of footwear 7a, 7b;
- the distance covered by the subject 3;
- the number of steps of at least one piece of footwear 7a, 7b.

Conveniently, the processing step provides for the processing of a motor characteristic of the type of at least one variable motor characteristic, i.e., the variation of one of the instantaneous motor characteristics in different instants of time.

In particular, the step of attribution or derivation comprises at least one step of indication of one or more of the motor characteristics to which to attribute to or from which to derive the attributed or derived reference parameter.

Preferably, the phase of acquisition provides for the acquisition of at least one motor datum selected from the list comprising speed, acceleration, pressure and position.

Moreover, the phase of acquisition preferably provides for the acquisition of a plurality of motor data.

Advantageously, the method comprises a verification phase of the detection of the FOG episode to confirm the occurrence of the latter, the emission phase being carried out as a result of the confirmation of the occurrence of the detected FOG episode.

Conveniently, the acquisition phase comprises at least the steps of:
- acquisition of at least one motor datum relating to a first piece of footwear 7a worn by the subject 3;
- acquisition of at least one motor datum relating to a second piece of footwear 7b worn by the subject 3.

This measure allows determining with accuracy the state of motion of each piece of footwear 7a, 7b, in order to accurately detect the occurrence of an FOG episode, thus reducing the risk of detecting false FOG episodes.

Suitably, the detection phase comprises at least one detection step of at least one of:
- at least one FOG episode depending on at least the motor datum relating to the first piece of footwear 7a;
- at least one FOG episode depending on at least the motor datum relating to the second piece of footwear 7b.

Preferably, the detection phase involves detecting an FOG episode depending on the acquired motor data.

Advantageously, the detection phase comprises at least one step of processing of at least one motor datum acquired to detect the occurrence of an FOG episode.

Preferably, the detection phase comprises a step of processing of a plurality of acquired motor data to detect the occurrence of an FOG episode.

In fact, the increase in the amount of acquired and processed data allows precisely detecting the occurrence of an FOG episode, thus reducing the risk of detecting false FOG episodes.

Advantageously, the verification phase comprises the verification of at least one of a detected FOG episode with reference to the first piece of footwear 7a, 7b and at least one detected FOG episode with reference to the second piece of footwear 7a, 7b to confirm the occurrence of the FOG episode.

Furthermore, the emission phase is carried out as a result of the confirmation of at least one of a detected FOG episode with reference to the first piece of footwear 7a, 7b and a detected FOG episode with reference to the second piece of footwear 7a, 7b.

In particular, according to a possible embodiment of the method, the emission phase is carried out as a result of the confirmation of one of an FOG episode detected with reference to the first piece of footwear 7a, 7b and an FOG episode detected with reference to the second piece of footwear 7a, 7b.

However, according to a further embodiment of the method, the emission phase is carried out as the result of the confirmation of an FOG episode detected with reference to the first piece of footwear 7a, 7b and an FOG episode detected with reference to the second piece of footwear 7a, 7b.

It has in practice been ascertained that the described invention achieves the intended object.

In particular, the fact is underlined that employing detection means separate from the acquisition means allows simplifying and making more flexible the use of the system, thus increasing, moreover, the precision of detection of the FOG episodes.

In addition, this solution makes it possible to reduce the exposure of the detection means to shocks and vibrations.

## Claims

1. System (1) for motor correction, particularly for overcoming FOG (Freezing of Gait) episodes, comprising:
- acquisition means (16) of at least one FOG-related motor datum of a subject (3) affected by FOG episodes, said acquisition means (16) being mounted on at least one piece of footwear (7a, 7b) worn by said subject (3);
- detection means (4), separate from said acquisition means (16), operationally connected to the latter and configured to detect at least one FOG episode depending on at least the detected motor datum;
- emission means (5) for emitting at least one sensory stimulus perceivable by said subject (3) as a result of the detection of the FOG episode and which allows the subject (3) to overcome such FOG episode, worn by said subject (3), and operationally connected to said detection means (4), the latter being configured to signal to said emission means (5) the detection of the FOG episode;
wherein said acquisition means (16) comprise at least one acquisition assembly (2a, 2b) provided with at least one sensor device (6) of the type selected from the list comprising a gyroscope, an accelerometer, a speedometer, a position locator, a pressure transducer, a magnetometer, and configured to acquire said at least one motor datum;
**characterized by** the fact that:
- said acquisition assembly (2a, 2b) comprises at least one detection unit (15) configured to detect at least one FOG episode depending on at least the motor datum;
- said detection means (4) are configured to verify the FOG episode detected by said detection unit (15) to confirm the occurrence thereof and to signal to said emission means (5) the detection of an FOG episode as a result of said confirmation, so that said emission means (5) emit the sensory stimulus as a result of verification and subsequent confirmation of the occurrence of the FOG episode;
and **characterized by** the fact that:
- said detection means (4) comprise at least one processing unit (9) of at least one motor characteristic processed depending on said at least one motor datum, said detection means (4) being configured to:
- compare at least one predefined reference value with at least one reference value attributed to or derived from one or more motor characteristics depending at least on the value of the latter;
- detect at least one FOG episode as a result of the comparison between said attributed or derived reference value and said at least one predefined reference value;
- said detection means (4) are configured to:
- process/provide said predefined reference value depending on a motor characteristic or multiple motor characteristics combined with each other taken into account to detect the occurrence of at least one FOG episode;
- attribute to or derive from the motor characteristic or from the combination of motor characteristics taken into account to detect the occurrence of at least one FOG episode said at least one attributed or derived reference value;
- said detection means (4) are configured to:
- identify said predefined reference value depending on each type or each combination of types of said motor characteristics taken into account to detect the occurrence of an FOG episode;
- compare said predefined reference value with said attributed or derived reference value of the motor characteristic taken into account to detect the occurrence of an FOG episode or with a combination of attributed or derived reference values of a combination of said motor characteristics taken into account to detect the occurrence of an FOG episode.

2. System (1) according to claim 1, **characterized by** the fact that said acquisition assembly (2a, 2b) comprises a plurality of said sensor devices (6).

3. System (1) according to one or more of the preceding claims, **characterized by** the fact that said acquisition means (16) comprise a pair of acquisition assemblies (2a, 2b), each of which is mounted on a corresponding footwear (7a, 7b) worn by said subject (3).

4. System (1) according to one or more of the preceding claims, **characterized by** the fact that said detection means (4) are configured to attribute to or derive from a linear combination of a plurality of motor characteristics said attributed or derived reference value.

5. System (1) according to one or more of the preceding claims, **characterized by** the fact that it comprises at least one of:
- said at least one footwear (7a, 7b) worn by said subject (3);
- at least one insole (13) insertable inside a corresponding footwear (7a, 7b) worn by said subject (3), at least one of either said acquisition means (16), detection means (4) or said emission means (5) being mounted on said insole (13).

6. System (1) according to one or more of the preceding claims, **characterized by** the fact that:
- said at least one footwear (7a, 7b) comprises at least one outsole (14);
- said detection means (4) are associated with said outsole (14);
- said acquisition assembly (2a, 2b) is associated with said outsole (14) and comprises at least one sensor device (6) of the type selected from the list comprising a gyroscope, an accelerometer, a speedometer, a position locator, a magnetometer, and at least one sensor device (6) of the type of a pressure transducer;
- said emission means (5) are worn on the upper part of the body of the subject (3) starting from the waistline of the same.

## Patentansprüche

1. System (1) zur motorischen Korrektur, insbesondere zur Überwindung von FOG-Episoden (Freezing of Gait), umfassend:
- Erfassungsmittel (16) für mindestens eine FOG-bezogene motorische Messgröße eines von FOG-Episoden betroffenen Subjekts (3), wobei die Erfassungsmittel (16) an mindestens einem Stück Fußbekleidung (7a, 7b) des Subjekts (3) angebracht ist;
- von den Erfassungsmitteln (16) getrennte, in Wirkverbindung mit diesen stehende Detektionsmittel (4), die dazu ausgebildet sind, abhängig von zumindest der erfassten motorischen Messgröße mindestens eine FOG-Episode zu erkennen;
- Ausgabemittel (5) zum Ausgeben mindestens eines sensorischen Reizes als Folge der Erkennung der FOG-Episode, der von dem Subjekt (3) wahrgenommen werden kann und es dem Subjekt (3) ermöglicht, eine solche FOG-Episode zu überwinden, die vom Subjekt (3) getragen werden und in Wirkverbindung mit den Detektionsmitteln (4) stehen, wobei letztere dazu ausgebildet sind, den Ausgabemitteln (5) die Erkennung der FOG-Episode zu signalisieren;
wobei die Erfassungsmittel (16) mindestens eine Erfassungsbaugruppe (2a, 2b) umfassen, die mit mindestens einer Sensoreinrichtung (6) ausgestattet ist, deren Typ aus der Liste ausgewählt ist, die ein Gyroskop, einen Beschleunigungsmesser, einen Geschwindigkeitsmesser, einen Positionsbestimmer, einen Druckwandler und ein Magnetometer umfasst, und die dazu ausgebildet ist, die mindestens eine motorische Messgröße zu erfassen;
**dadurch gekennzeichnet, dass**:
- die Erfassungsbaugruppe (2a, 2b) mindestens eine Detektionseinheit (15) umfasst, die dazu ausgebildet ist, mindestens eine FOG-Episode in Abhängigkeit von mindestens einer motorischen Messgröße zu erfassen;
- die Detektionsmittel (4) dazu ausgebildet sind, die von der Detektionseinheit (15) erkannte FOG-Episode zu verifizieren, um deren Auftreten zu bestätigen, und den Ausgabemitteln (5) die Erkennung einer FOG-Episode als Ergebnis dieser Bestätigung zu signalisieren, sodass die Ausgabemittel (5) den sensorischen Reiz als Ergebnis der Verifizierung und der anschließenden Bestätigung des Auftretens der FOG-Episode ausgeben;
und **dadurch gekennzeichnet, dass**:
- die Detektionsmittel (4) mindestens eine Verarbeitungseinheit (9) für mindestens eine motorische Eigenschaft umfassen, die in Abhängigkeit von der mindestens einen motorischen Messgröße verarbeitet wird, wobei die Detektionsmittel (4) dazu ausgebildet sind:
- mindestens einen vordefinierten Referenzwert mit mindestens einem Referenzwert zu vergleichen, der einer oder mehreren motorischen Eigenschaften zugeordnet ist oder aus diesen abgeleitet ist, in Abhängigkeit zumindest vom Wert der letzteren;
- mindestens eine FOG-Episode als Ergebnis des Vergleichs zwischen dem zugeordneten oder abgeleiteten Referenzwert und dem mindestens einen vordefinierten Referenzwert zu erkennen;
- die Detektionsmittel (4) dazu ausgebildet sind,
- den vordefinierten Referenzwert in Abhängigkeit von einer motorischen Eigenschaft oder mehreren miteinander kombinierten motorischen Eigenschaften zu verarbeiten/bereitzustellen, die zur Erkennung des Auftretens mindestens einer FOG-Episode berücksichtigt werden;
- den mindestens einen zugeordneten oder abgeleiteten Referenzwert der motorischen Eigenschaft oder der Kombination von motorischen Eigenschaften zuzuordnen oder daraus abzuleiten, die zur Erkennung des Auftretens mindestens einer FOG-Episode berücksichtigt werden;
- die Detektionsmittel (4) dazu ausgebildet sind,
- den vordefinierten Referenzwert in Abhängigkeit von jedem Typ oder jeder Kombination von Typen der motorischen Eigenschaften zu identifizieren, die zur Erkennung des Auftretens einer FOG-Episode berücksichtigt werden;
- den vordefinierten Referenzwert mit dem zugeordneten oder abgeleiteten Referenzwert der motorischen Eigenschaft, die zur Erkennung des Auftretens einer FOG-Episode berücksichtigt wird, oder mit einer Kombination aus zugeordneten oder abgeleiteten Referenzwerten einer Kombination der zur Erkennung des Auftretens einer FOG-Episode berücksichtigten motorischen Eigenschaften zu vergleichen.

2. System (1) nach Anspruch 1, **dadurch gekennzeichnet, dass** die Erfassungsbaugruppe (2a, 2b) eine Mehrzahl der Sensoreinrichtungen (6) umfasst.

3. System (1) nach einem oder mehreren der vorstehenden Ansprüche, **dadurch gekennzeichnet, dass** die Erfassungsmittel (16) ein Paar von Erfassungsbaugruppen (2a, 2b) umfassen, von denen jede an einem entsprechenden, vom Subjekt (3) getragenen Stück Fußbekleidung (7a, 7b) angebracht ist.

4. System (1) nach einem oder mehreren der vorstehenden Ansprüche, **dadurch gekennzeichnet, dass** die Detektionsmittel (4) dazu ausgebildet sind, den zugeordneten oder abgeleiteten Referenzwert einer linearen Kombination aus mehreren motorischen Eigenschaften zuordnen oder daraus abzuleiten.

5. System (1) nach einem oder mehreren der vorstehenden Ansprüche, **dadurch gekennzeichnet, dass** es mindestens eines der folgenden Elemente umfasst:
- das mindestens eine vom Subjekt (3) getragene Stück Fußbekleidung (7a, 7b);
- mindestens eine Einlegesohle (13), die in ein entsprechendes, vom Subjekt (3) getragenes Stück Fußbekleidung (7a, 7b) eingesetzt werden kann, wobei mindestens eines der Erfassungsmittel (16), der Detektionsmittel (4) oder der Ausgabemittel (5) an der Einlegesohle (13) angebracht ist.

6. System (1) gemäß einem oder mehreren der vorstehenden Ansprüche, **dadurch gekennzeichnet, dass**:
- das mindestens eine Stück Fußbekleidung (7a, 7b) mindestens eine Außensohle (14) umfasst;
- die Detektionsmittel (4) der Außensohle (14) zugeordnet sind;
- die Erfassungsbaugruppe (2a, 2b) der Außensohle (14) zugeordnet ist und mindestens eine Sensoreinrichtung (6) umfasst, deren Typ aus einer Liste ausgewählt ist, die ein Gyroskop, einen Beschleunigungsmesser, einen Geschwindigkeitsmesser, einen Positionsbestimmer, ein Magnetometer sowie mindestens eine Sensoreinrichtung (6) vom Typ eines Druckwandlers umfasst;
- die Ausgabemittel (5) am Oberkörper des Subjekts (3) ab der Taille desselben getragen werden.

## Revendications

1. - Système (1) de correction motrice, en particulier pour surmonter des épisodes de FOG (blocage brutal à l'initiation de la marche), comprenant :
- des moyens d'acquisition (16) d'au moins une donnée motrice associée au FOG d'un sujet (3) atteint d'épisodes de FOG, lesdits moyens d'acquisition (16) étant montés sur au moins une chaussure (7a, 7b) portée par ledit sujet (3) ;
- des moyens de détection (4), distincts desdits moyens d'acquisition (16), reliés de manière fonctionnelle à ces derniers et configurés pour détecter au moins un épisode de FOG en fonction d'au moins la donnée motrice détectée ;
- des moyens d'émission (5) pour émettre au moins un stimulus sensoriel perceptible par ledit sujet (3) suite à la détection de l'épisode de FOG et qui permet au sujet (3) de surmonter un tel épisode de FOG, portés par ledit sujet (3) et reliés de manière fonctionnelle auxdits moyens de détection (4), ces derniers étant configurés pour signaler auxdits moyens d'émission (5) la détection de l'épisode de FOG ;
dans lequel lesdits moyens d'acquisition (16) comprennent au moins un ensemble d'acquisition (2a, 2b) comportant au moins un dispositif de capteur (6) du type choisi parmi la liste comprenant un gyroscope, un accéléromètre, un compteur de vitesse, un localisateur de position, un transducteur de pression, un magnétomètre, et configuré pour acquérir ladite au moins une donnée motrice;
**caractérisé par le fait que** :
- ledit ensemble d'acquisition (2a, 2b) comprend au moins une unité de détection (15) configurée pour détecter au moins un épisode de FOG en fonction d'au moins la donnée motrice ;
- lesdits moyens de détection (4) sont configurés pour vérifier l'épisode de FOG détecté par ladite unité de détection (15) afin de confirmer la survenue de celui-ci et de signaler auxdits moyens d'émission (5) la détection d'un épisode de FOG suite à ladite confirmation, de sorte que lesdits moyens d'émission (5) émettent le stimulus sensoriel suite à la vérification et à la confirmation ultérieure de la survenue de l'épisode de FOG ; et
**caractérisé par le fait que** :
- lesdits moyens de détection (4) comprennent au moins une unité de traitement (9) d'au moins une caractéristique motrice traitée en fonction de ladite au moins une donnée motrice, lesdits moyens de détection (4) étant configurés pour :
- comparer au moins une valeur de référence prédéfinie à au moins une valeur de référence affectée à ou dérivée d'une ou plusieurs caractéristiques motrices en fonction au moins de la valeur de ces dernières ;
- détecter au moins un épisode de FOG suite à la comparaison entre ladite valeur de référence affectée ou dérivée et ladite au moins une valeur de référence prédéfinie ;
- lesdits moyens de détection (4) sont configurés pour :
- traiter/fournir ladite valeur de référence prédéfinie en fonction d'une caractéristique motrice ou de plusieurs caractéristiques motrices combinées les unes avec les autres prises en compte pour détecter la survenue d'au moins un épisode de FOG ;
- affecter à ou dériver de la caractéristique motrice ou de la combinaison de caractéristiques motrices prises en compte pour détecter la survenue d'au moins un épisode de FOG ladite au moins une valeur de référence affectée ou dérivée ;
- lesdits moyens de détection (4) sont configurés pour :
- identifier ladite valeur de référence prédéfinie en fonction de chaque type ou de chaque combinaison de types desdites caractéristiques motrices prises en compte pour détecter la survenue d'un épisode de FOG ;
- comparer ladite valeur de référence prédéfinie à ladite valeur de référence affectée ou dérivée de la caractéristique motrice prise en compte pour détecter la survenue d'un épisode de FOG ou à une combinaison de valeurs de référence affectées ou dérivées d'une combinaison desdites caractéristiques motrices prises en compte pour détecter la survenue d'un épisode de FOG.

2. - Système (1) selon la revendication 1, **caractérisé par le fait que** ledit ensemble d'acquisition (2a, 2b) comprend une pluralité desdits dispositifs de capteur (6).

3. - Système (1) selon une ou plusieurs des revendications précédentes, **caractérisé par le fait que** lesdits moyens d'acquisition (16) comprennent une paire d'ensembles d'acquisition (2a, 2b), dont chacun est monté sur une chaussure correspondante (7a, 7b) portée par ledit sujet (3).

4. - Système (1) selon une ou plusieurs des revendications précédentes, **caractérisé par le fait que** lesdits moyens de détection (4) sont configurés pour affecter à ou dériver d'une combinaison linéaire d'une pluralité de caractéristiques motrices ladite valeur de référence affectée ou dérivée.

5. - Système (1) selon une ou plusieurs des revendications précédentes, **caractérisé par le fait qu'**il comprend au moins l'un parmi :
- ladite au moins une chaussure (7a, 7b) portée par ledit sujet (3) ;
- au moins une semelle intérieure (13) apte à être insérée à l'intérieur d'une chaussure correspondante (7a, 7b) portée par ledit sujet (3), au moins l'un parmi lesdits moyens d'acquisition (16), les moyens de détection (4) ou lesdits moyens d'émission (5) étant monté sur ladite semelle intérieure (13).

6. - Système (1) selon une ou plusieurs des revendications précédentes, **caractérisé par le fait que** :
- ladite au moins une chaussure (7a, 7b) comprend au moins une semelle extérieure (14) ;
- lesdits moyens de détection (4) sont associés à ladite semelle extérieure (14) ;
- ledit ensemble d'acquisition (2a, 2b) est associé à ladite semelle extérieure (14) et comprend au moins un dispositif de capteur (6) du type choisi parmi la liste comprenant un gyroscope, un accéléromètre, un compteur de vitesse, un localisateur de position, un magnétomètre, et au moins un dispositif de capteur (6) du type transducteur de pression ;
- lesdits moyens d'émission (5) sont portés sur la partie supérieure du corps du sujet (3), à partir de la taille de celui-ci.
